# EUROPÄISCHE PATENTSCHRIFT

(11) **EP 1 088 531 B1**
(45) Veröffentlichungstag und Bekanntmachung des Hinweises auf die Patenterteilung: **31.03.2004**
(21) Anmeldenummer: 99118860.8
(22) Anmeldetag: 24.09.1999
(51) Int. Cl.: A61F 2/38

(54) **Gelenkendoprothese**
Joint prosthesis
Prothèse articulaire

(43) Veröffentlichungstag der Anmeldung: 04.04.2001
(73) Patentinhaber: WALDEMAR LINK GmbH & Co. KG, 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 339 530
- EP-A- 0 376 658
- WO-A-98/04215
- FR-A- 2 673 832
- US-A- 5 405 403
- US-A- 5 653 764

## Beschreibung

Gelenkendoprothesen weisen außer denjenigen Komponenten, die zur Nachbildung der Gelenkfunktion dienen, Teile zur Verankerung am Knochen auf. Wenn für eine Prothese unterschiedliche Verankerungsteile bereitgehalten werden, wird der jeweils gewählte Verankerungsteil über eine Kupplungseinrichtung mit einer zugehörigen Gelenkkomponente verbunden. Bewährt haben sich dafür Konuskupplungen, die aus einem Konuszapfen und einer Konushülse bestehen, die mit gleichem, geringem Konuswinkel von beispielsweise 5°, ausgebildet sind und in der Regel hinreichend fest ineinander haften, sobald sie unter Ausübung eines Stoßes zusammengefügt wurden. Wenn keine wesentlichen Kräfte auftreten, die in Richtung der Trennung der Kupplung wirken, sind weitere Sicherungsmittel nicht notwendig. Dies gilt beispielsweise für die Befestigung von Hüftgelenkköpfen auf den zugehörigen Schaftteilen (US-A 5336268). Wenn man mit beträchtlichen Lösekräften rechnen muß, versieht man die Konuskupplung mit einer Sicherungsschraube (EP-A 474015). Beispielsweise kann im Konuszapfen eine Madenschraube enthalten sein, deren kegelige Spitze in Eingriff mit einer exzentrisch dazu liegenden Bohrung der Konushülse geschraubt wird (EP-A 474015, Fig. 3; WO 9 804 215).

Ferner ist es bekannt (US-A 5405403) eine Konusverbindung mit einer Drehsicherung zu versehen, um eine Relativdrehung.zwischen den Konusflächen zu vermeiden, weil diese zur Korrosion führen könnte.

Eine Sicherungsschraube verhindert nicht nur eine translatorische Bewegung der gekuppelten Teile in Achsrichtung, sondern auch eine Rotationsbewegung. Herkömmlicherweise erfüllt eine Sicherungsschraube daher auch die Aufgaben einer Drehsicherung.

Die Erfindung hat erkannt, daß durch die Kombination einer Sicherungsschraube mit einer Drehsicherung beträchtliche Vorteile erzielt werden, wenn die Sicherungsschraube über eine kegelige Spitze mit einer exzentrisch dazu liegenden Bohrung zusammenwirkt. Bei Verwendung einer solchen Sicherungsschraube ohne Drehsicherung, wie es dem Stand der Technik entspricht, ist bei der Montage nicht gewährleistet, daß die Kegelspitze der Sicherungsschraube genau mit demjenigen Punkt der Bohrungskante zusammenwirkt, der dem dickeren Ende der Konuskupplung am nächsten liegt. Nur dann nämlich tritt eine exakte Drehsicherung durch die Sicherungsschraube ein. Liegt der Kontaktpunkt jedoch ein wenig seitlich neben diesem idealen Punkt des Zusammenwirkens, sind Mikro-Rotationsbewegungen möglich. Die erfindungsgemäße Kombination sorgt jedoch dafür, daß ein bestimmter Kontaktpunkt zwischen der Kegelspitze, der Sicherungsschraube und der zugehörigen Bohrung festgelegt wird. Dadurch werden nicht nur die genannten Mikro-Bewegungen ausgeschlossen, sondern es wird auch vermieden, daß die Sicherungsschraube durch Relativbewegungen gegenüber der mit ihr zusammenwirkenden Bohrungskante Verschleiß erleidet, verdreht wird und ggf. ihre sichernde Funktion ganz oder teilweise verliert.

Von besonderer Bedeutung ist dies dann, wenn die Sicherungsschraube mit einer Sollbruchstelle versehen ist (EP-A 915686 = WO 9804215). Durch die Drehsicherung wird somit bewirkt, daß die Sicherungsschraube ihre Funktion in der gewünschten Weise erfüllen kann.

Zweckmäßigerweise werden zur Ausbildung dieser Drehsicherung Vorsprünge und Ausnehmungen an den beiden Kupplungsteilen gebildet, die über achsparallele Flanken zusammenwirken. Beispielsweise kann vom Rand der Konushülse wenigstens ein Zapfen achsparallel vorspringen und in eine entsprechend geformte Nut eingreifen, die an der Wurzel des Konuszapfens vorgesehen ist. Umgekehrt ist es auch möglich, den Rand der Konushülse mit mindestens einem Einschnitt zu versehen, in den ein am Konuszapfen vorgesehener Querstift eingreift.

Die Erfindung wird im folgenden näher unter Bezugnahme auf die Zeichnung erläutert, die vorteilhafte Ausführungsbeispiele veranschaulicht. Es zeigen:
- Fig. 1: die Seitenansicht einer Knieprothese und
- Fig. 2 u. 3.: eine Seitenansicht und eine Schnittansicht der Konuskupplung.

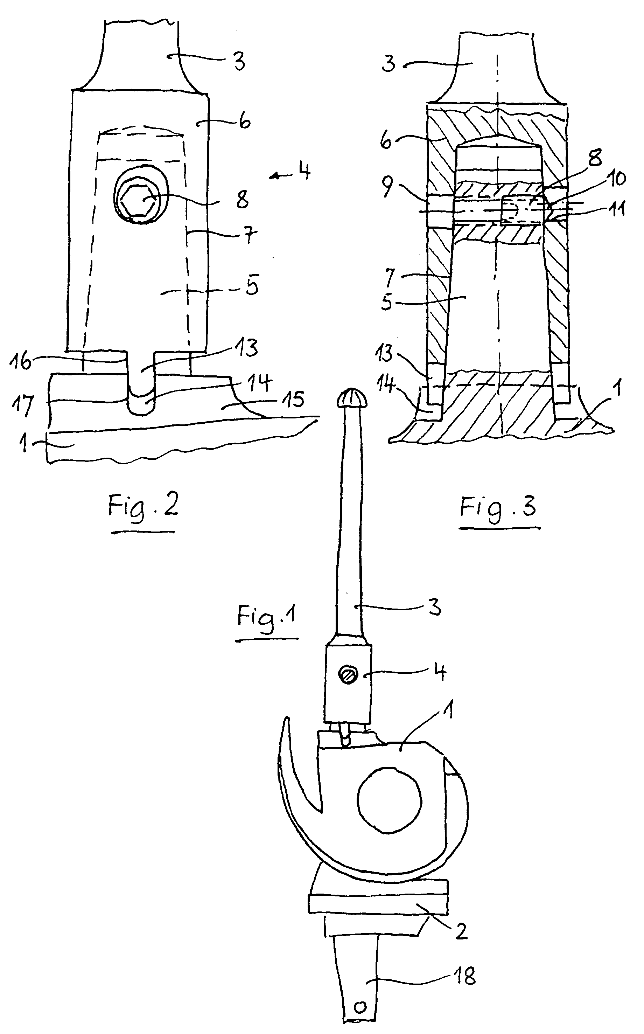

Die in Fig. 1 gezeigte Knieprothese besitzt eine femorale Komponente 1 und eine tibiale Komponente 2. Die femorale Komponente ist gezeigt in Verbindung mit einem Schaft 3, der in der Höhlung des Oberschenkelknochens zu verankern ist. Er ist mit der Femurkomponente 1 über eine Konuskupplung 4 verbunden. Die Tibiakomponente 2 ist gleichfalls mit einem nicht gezeigten Verankerungsteil zu verbinden und besitzt dafür als Teil einer Konuskupplung einen Konuszapfen 5.

Die Konuskupplung besteht aus einem Konuszapfen 5 und einer Konushülse 6, die über Flächen 7 gleicher Konizität großflächig in Berührung stehen. Der Konuswinkel dieser Flächen (Winkel zwischen diametral gegenüberliegenden Mantellinien) liegt beispielsweise in der Größenordnung von 5°. Er ist so bemessen, daß die Kupplungsteile 5 und 6, nachdem sie kraftvoll (beispielsweise mittels eines Schlags) zusammengefügt wurden, nur mit beträchtlicher Kraft wieder zu trennen sind und auch nur gegen beträchtlichen Reibwiderstand relativ zueinander verdreht werden können. In manchen Fällen kann die Festigkeit der Kupplung in Frage gestellt sein, nämlich wenn sie bei der Implantation nicht hinreichend stark zusammengefügt wurden oder wenn sie durch eine anomal hohe Lösekraft während des Gebrauchs gelockert wurden. In dem Beispiel ist deshalb eine Madenschraube 8 im Kupplungszapfen 5 vorgesehen, deren Schlüsselsechskant durch eine Bohrung 9 auf der einen Seite der Konushülse 6 zugänglich ist und deren Kegelspitze 10 mit der Kante 11 einer Bohrung 12 auf der anderen Seite der Konushülse 6 zusammenwirkt.

Um die Schraube 8 von der Belastung durch Rotationskräfte zu befreien bzw. um im Falle des Fehlens der Schraube 8 die Drehsicherheit der Kupplung zu gewährleisten, ist die erfindungsgemäße Drehsicherung vorgesehen, die aus zwei achsparallelen Zapfen 13 am Rand der Konushülse 6 und entsprechenden Nuten 14 in dem Kragen 15 besteht, von dem der Konuszapfen 5 vorspringt. Die Zapfen 13 und die Nuten 14 sind jeweils durch ein Paar paralleler Flanken 16 bzw. 17 begrenzt, wobei die Weite der Nut 14 nicht oder kaum größer ist als die Breite der Zapfen 13.

Zweckmäßigerweise sind zwei einander etwa gegenüberliegende Drehsicherungen 13, 14 vorgesehen.

Die Bohrungen 9, 12 der Schraubsicherung fluchten nicht miteinander. Es muß deshalb dafür gesorgt werden, daß die Konushülse 6 in einer bestimmten Drehstellung auf den Zapfen 5 aufgesetzt wird, in welcher die Schraubenspitze 10 richtig mit der Bohrung 12 zusammenwirkt. Dies kann dadurch gewährleistet werden, daß die Drehsicherung oder Drehsicherungen so angeordnet wird/werden, daß diese bestimmte Drehstellung zustande kommt. Dies ist zwangsläufig der Fall, wenn lediglich eine Drehsicherung 13, 14 verwendet wird oder wenn zwei Drehsicherungen nicht genau diametral gegenüberliegend positioniert werden.

Bei dem Verankerungsteil muß es sich nicht um einen Knochenschaft handeln; es kann auch die Stange sein, die im Falle einer Totalprothese eines von zwei Gelenken eingeschlossenen Knochenstücks eine Komponente des einen Gelenks mit einer Komponente des anderen Gelenks verbindet.

## Patentansprüche

1. Gelenkendoprothese mit einem Verankerungsteil und einer Konuskupplung zwischen einem Gelenkteil und dem Verankerungsteil, die mit einer Sicherungsschraube (8) versehen ist, deren Kegelspitze (10) mit der Kante (11) einer zu der Sicherungsschraube (8) exzentrischen Bohrung (12) zusammenwirkt, **dadurch gekennzeichnet, daß** die Konuskupplung (4) zusätzlich zu der Sicherungsschraube (8) eine Drehsicherung (13, 14) aufweist.

2. Gelenkendoprothese nach Anspruch 1, **dadurch gekennzeichnet, daß** die Drehsicherung von je einem Vorsprung (13) bzw. einer Ausnehmung an beiden Teilen gebildet ist; die über achsparallele Flanken (16, 17) zusammenwirken.

3. Gelenkendoprothese nach Anspruch 2, **dadurch gekennzeichnet, daß** vom Rand der Konushülse (6) wenigstens ein Zapfen (13) vorspringt und an dem Kragen (15) des Konuszapfens (5) eine entsprechende Nut (14) vorgesehen ist.

4. Gelenkendoprothese nach Anspruch 2, **dadurch gekennzeichnet, daß** der Rand der Konushülse (6) mindestens einen Einschnitt aufweist und am Konuszapfen (5) ein entsprechender Querstift vorgesehen ist.

## Claims

1. A joint endoprosthesis with an anchoring part and a cone coupling between a joint part and the anchoring part, which is provided with a securing screw (8) whose conical tip (10) co-operates with the edge (11) of a bore (12) which is eccentric relative to the securing screw (8), **characterised in that** in addition to the securing screw the cone coupling (4) has a means (13,14) for preventing relative rotation.

2. A joint endoprosthesis according to Claim 1, **characterised in that** the means for preventing relative rotation is formed by a projection (13) and a recess on respective parts, which co-operate via axially parallel flanks (16,17).

3. A joint endoprosthesis according to Claim 2, **characterised in that** at least one peg (13) projects from the edge of the cone socket (6) and a corresponding groove (14) is provided on the collar (15) of the conical projection (5) .

4. A joint endoprosthesis according to Claim 2, **characterised in that** the edge of the cone socket(6) has at least one indentation and a corresponding transverse pin is provided on the conical projection (5).

## Revendications

1. Endoprothèse d'articulation comportant une partie d'ancrage et un accouplement à cône entre une partie d'articulation et la partie d'ancrage, lequel accouplement est pourvu d'un vis de blocage (8) dont la pointe conique (10) coopère avec le bord (11) d'un perçage (12) excentré par rapport à la vis de blocage (8), **caractérisée en ce que** l'accouplement à cône (4) comporte un blocage en rotation (13, 14) en plus de la vis de blocage (8).

2. Endoprothèse d'articulation selon la revendication 1, **caractérisée en ce que** le blocage en rotation est formé par une saillie (13) ou un évidement sur chacune des deux pièces qui coopèrent par des flancs (16, 17) parallèles à l'axe.

3. Endoprothèse d'articulation selon la revendication 2, **caractérisée en ce que** du bord de la douille à cône (6) fait saillie au moins un tenon (13) et sur le collet (15) de la tige de cône (5) est prévue une rainure (14) correspondante.

4. Endoprothèse d'articulation selon la revendication 2, **caractérisée en ce que** le bord de la douille à cône (6) présente au moins une entaille et sur la tige de cône (5) est prévue un broche transversale correspondante.
